# EUROPEAN PATENT SPECIFICATION

(11) **EP 2 223 122 B1**
(45) Date of publication and mention of the grant of the patent: **31.08.2011**
(21) Application number: 08863184.1
(22) Date of filing: 18.12.2008
(51) Int. Cl.: G01N 33/94

(54) **ENDOGENOUS MORPHINE OR A NATURALLY OCCURING METABOLITE THEREOF AS A MARKER FOR SEPSIS**
ENDOGENES MORPHIN ODER NATÜRLICH VORKOMMENDER METABOLIT DAVON ALS MARKER FÜR SEPSIS
MORPHINE ENDOGÈNE OU MÉTABOLITE D'ORIGINE NATURELLE DE CELLE-CI EN TANT QUE MARQUEUR POUR LA SÉPTICÉMIE

(30) Priority: 18.12.2007 EP 07301692
(43) Date of publication of application: 01.09.2010
(73) Proprietor: INSERM (Institut National de la Santé et de la Recherche Médicale), 75654 Paris Cedex 13 (FR); Justus-Liebig Universität, 35390 Giessen (DE)
(72) Inventor: GOUMON, Yannick, F-67084 Strasbourg (FR); AUNIS, Dominique, F-67084 Strasbourg (FR); METZ-BOUTIGUE, Marie-Hélène, F-67084 Strasbourg (FR); SCHNEIDER, Francis, F-67098 Strasbourg (FR); WELTERS, Ingeborg, 35396 Giessen (DE)
(74) Representative: Cabinet Plasseraud
(86) International application number: PCT/EP2008/067907
(87) International publication number: WO 2009/077593

(56) References cited:
- KUMAR ET AL: "Basic science of sepsis" SURGERY, MEDICINE PUBLISHING, ABINGTON. / ELSEVIER IMPRINT, GB, vol. 23, no. 8, 1 August 2005 (2005-08-01), pages 272-277, XP005768856 ISSN: 0263-9319
- PANTALEO G ET AL: "Immunopathogenesis of HIV infection." ANNUAL REVIEW OF MICROBIOLOGY 1996, vol. 50, 1996, pages 825-854, XP002478095 ISSN: 0066-4227
- GREENWOOD B M ET AL: "Malaria" LANCET THE, LANCET LIMITED. LONDON, GB, vol. 365, no. 9469, 23 April 2005 (2005-04-23), pages 1487-1498, XP004861415 ISSN: 0140-6736
- "Resumes des communications orales et des posters de la reunion scientifique Paris, 22-24 janvier 1997" REANIMATION URGENCES, EDITIONS SCIENTIFIQUES ET MEDICALES ELSEVIER, vol. 5, no. 6, 1996, pages 741-837, XP004953387 ISSN: 1164-6756
- STEFANO, G. B.: "Advances in Endogenous Morphine" MEDICAL SCIENCE MONITOR, vol. 11, no. 5, 2005, XP008090863
- GLATTARD E ET AL: "Rethinking the opiate system? Morphine and morphine-6-glucuronide as new endocrine and neuroendocrine mediators" MEDICAL SCIENCE MONITOR, MEDISCIENCE PUBLIKACJE NAUKOWE, WARZAW, PL, vol. 12, no. 6, June 2006 (2006-06), pages SR25-SR27, XP008090818 ISSN: 1234-1010

## Description

### FIELD OF THE INVENTION

The invention relates to a method for detecting sepsis in a patient.

### BACKGROUND OF THE INVENTION

Infection is the detrimental colonization of a host organism by a foreign species. The infectious agent, or pathogen, interferes with the normal functioning of the host and can lead to dysfunction of the infected tissue, fluid, organ or organism, and even to death. Infection can be caused by a variety of pathogens, including bacteria, parasites, fungi, viruses, prions, and viroids.

Diagnosis of infectious disease can involve the identification of the infectious agent, either directly or indirectly.

Direct identification of a pathogen can be carried out after a microbial culture. A sample taken from potentially diseased tissue or fluid is tested for the presence of an infectious agent able to grow within a specific growth medium. For example, most pathogenic bacteria are easily grown on nutrient agar plates where they will form colonies. The size, color, shape and form of a colony is characteristic of the bacterial species, its specific genetic makeup (its strain), and the environment which supports its growth. Other ingredients are often added to the plate to facilitate identification. Plates may contain substances that permit the growth of some bacteria and not others, or that change color in response to certain bacteria and not others. Microbial culture may also be used in the identification of viruses: the medium in this case being cells grown in culture that the virus can infect, and then alter or kill. In the case of viral identification, a region of dead cells results from viral growth, and is called a "plaque". Eukaryotic parasites may also be grown in culture as a means of identifying a particular agent.

Direct identification of infectious agents can also rely on biochemical tests such as the detection of metabolic or enzymatic products characteristic of a particular infectious agent. For example, in the case of bloodstream infection by bacteria, bacterial toxins can be secreted into the bloodstream and readily measured.

Immunodiagnostic methods are highly sensitive, specific and often extremely rapid tests used to identify microorganisms. These tests are based upon the ability of an antibody to bind specifically to an antigen. The antigen, usually a protein or carbohydrate made by an infectious agent, is bound by the antibody. This binding then sets off a chain of events that can be visibly obvious in various ways, dependent upon the test.

PCR-based methods can also be used to detect a pathogen if the genome of the pathogen is known. One can generate specific primers recognizing a specific genomic region of the pathogen and amplify this region from a sample obtained from a patient.

Indirect identification of a pathogen can be carried out by detecting, in a sample obtained from a patient, a molecule which is specifically induced by the pathogen. For example, many infectious diseases are diagnosed by the presence of specific antibodies against the pathogen in the bloodstream of the patient.

An alternative manner to detect infection which does not require the identification of the pathogen itself is to detect the organism's response to infection. Indeed, bloodstream infection usually results in an inflammatory response, as is the case in sepsis. Sepsis is an infection-induced syndrome characterized by a generalized inflammatory state and represents a frequent complication in the surgical patient (Tsiotou et al., 2005).

Sepsis assessment relies on various laboratory parameters and hemodynamic parameters. However, changes in body temperature, heart and respiratory rate and white cell count (the "SIRS" criteria) are not specific enough to identify infected patients. Among many biological parameters, measurement of lactate, central venous oxygen saturation (ScvO2), C-reactive protein (CRP) and procalcitonin (PCT) are of particular interest. (see Spapen et al. 2006 for review). Document US Pat. No 5,639,617 describes the quantification of PCT for assessing the course of sepsis Bensousan et al. (Bensousan et al., 1996) identify CRP, PCT and Interleukin 6 (IL6) as biomarkers in the diagnosis of sepsis.

Document US Application No 2004/0180396 discloses the use of other peptide prohormones as biomarkers in the diagnosis of sepsis.

To date, the diagnosis of sepsis relies on a panel of parameters, the determination of which is costly and time-consuming. Moreover, if sepsis is accompanied by clinical and laboratory signs of systemic inflammation, patients with inflammation caused by non-infectious causes may also present similar signs and symptoms (Reinhart et al 2006).

Thus there is still an existing need to develop an accurate and specific method for diagnosing sepsis which is independent of the infectious agent.

### SUMMARY OF THE INVENTION

In fulfilling this need, there is described a method for detecting sepsis in a patient, said method comprising detecting endogenous morphine or a naturally occurring metabolite thereof in a biological sample obtained from said patient.

The invention also relates to method for detecting sepsis, in a patient, said method comprising measuring the concentration of endogenous morphine or a naturally occurring metabolite thereof in a biological sample obtained from said patient.

The invention also relates to the use of endogenous morphine or a naturally occurring metabolite thereof as a marker of sepsis, in a patient.

### DETAILED DESCRIPTION OF THE INVENTION

### Definitions:

As used herein, the term "infection" refers to the pathological process caused by the invasion of normally sterile tissue or fluid or body cavity by pathogenic or potentially pathogenic micro-organisms.

As used herein, the term "bloodstream infection" refers to a disease wherein the infectious agent is present in the bloodstream of the patient.

As used herein, the terms "sepsis", "severe sepsis" and "septic shock" are used to identify the continuum of the clinical response to infection. Patients with sepsis present evidences of infection and clinical manifestations of inflammation. Patients with severe sepsis develop hypoperfusion with organ dysfunction. Septic shock is manifested by hypoperfusion and persistent hypotension.

The term "endogenous morphine" as used herein has its general meaning in the art (see, for example, Stefano et al., 2000). Endogenous morphine and naturally occurring metabolites thereof were identified in numerous mammalian tissues (Goumon et al., 2005; Goumon et al., 2006). Examples of naturally occurring metabolites of morphine are morphine-6-glucuronide (M6G) and morphine-3-glucuronide (M3G).

In a preferred embodiment of the invention, "a naturally occurring metabolite of morphine" is M6G.

The term "anti-morphine antibody" refers to an antibody or a fragment thereof which recognizes morphine or a naturally occurring metabolite thereof.

The term "anti-CRP" antibody refers to an antibody or a fragment thereof which recognizes C-reactive protein (CRP).

The term "anti-procalcitonin" antibody refers to an antibody or a fragment thereof which recognizes procalcitonin.

The term "patient" as used herein denotes a mammal such as a rodent, a feline, a canine and a primate. Preferably, a patient according to the invention is a human.

### Diagnostic methods and kits:

The present invention relates to a method for detecting sepsis in a patient, said method comprising detecting endogenous morphine or a naturally occurring metabolite thereof in a biological sample obtained from said patient.

The present invention relates to a method for detecting sepsis in a patient, said method comprising measuring the concentration of endogenous morphine or a naturally occurring metabolite thereof in a biological sample obtained from said patient.

Examples of biological samples suitable for the method of the invention include, but are not limited to whole blood, serum, plasma, urine, saliva and cerebrospinal fluid. Typically, the biological sample is whole blood, serum, plasma, or urine. Typically, the biological sample is serum.

As the skilled person will appreciate, when the method comprises the detection of endogenous morphine or a naturally occurring metabolite thereof in a biological sample, the biological sample will be one which is devoid of endogenous morphine or a naturally occurring metabolite thereof under normal conditions, i.e. in a patient who does not suffer from infection.

As the skilled person will appreciate, if the patient has received a treatment comprising morphine or a molecule which can be metabolized into morphine such as codeine, this can complicate the determination of the concentration of endogenous morphine or a naturally occurring metabolite thereof. In a preferred embodiment of the invention, the patient has not been treated with any exogenous morphine or molecule which can be metabolized by the patient into morphine, such as codeine.

Once the biological sample from the patient is prepared, the concentration of endogenous morphine or a naturally occurring metabolite thereof may be measured by any known method in the art.

In a particular embodiment, such methods comprise contacting the biological sample with a binding partner capable of selectively interacting with endogenous morphine or a naturally occurring metabolite thereof present in the biological sample. The binding partner may be an antibody that may be polyclonal or monoclonal, preferably monoclonal. In another embodiment, the binding partner may be an aptamer.

Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.

Monoclonal antibodies of the invention or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). For example, document TW 526269 discloses monoclonal antibodies against morphine. Alternatively, techniques described for the production of single chain antibodies (see e.g. U.S. Pat. No. 4,946,778) can be adapted to produce anti-morphine, single chain antibodies. Antibodies useful in practicing the present invention also include anti-morphine fragments including but not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed to allow rapid identification of fragments having the desired specificity to morphine. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.

In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996). US Patent Application No. 2003/0224435 discloses the use of aptamers for the detection and quantification of various drugs, including morphine, in a biological sample.

The binding partners of the invention such as antibodies or aptamers, may be labelled with a detectable molecule or substance, such as a fluorescent molecule, an enzyme able to produce a coloured product, a radioactive molecule or any others labels known in the art. Labels are known in the art that generally provide (either directly or indirectly) a signal.

As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5), to the antibody or aptamer, as well as indirect labelling of the probe or antibody (e.g., horseradish peroxidise, HRP) by reactivity with a detectable substance. An antibody or aptamer of the invention may be labelled with a radioactive molecule by any method known in the art. For example radioactive molecules include but are not limited radioactive atom for scintigraphic studies such as I123, I124, In111, Re186, Re188.

The aforementioned assays generally involve the bounding of the binding partner (i.e., Antibody or aptamer) in a solid support. Solid supports which can be used in the practice of the invention include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of anti-morphine antibodies. A biological sample containing or suspected of containing morphine or a naturally occurring metabolite thereof is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art. An alternative method of ELISA involves the addition of a biological sample containing or suspected of containing morphine or a naturally occurring metabolites thereof to the coated wells in addition to a known amount of labelled morphine or metabolite thereof. After a period of incubation sufficient to allow the formation of antibody-antigen complexes and the competition between labelled and non-labelled naturally occurring molecules, the plate(s) can be washed to remove unbound moieties. The intensity of the label obtained by the binding of, for example HRP-conjugated morphine or metabolite thereof, is detected using methods well known in the art. In this configuration, that is more sensitive that common ELISA, decrease of the label is in relation with the amount of natural molecules present in the sample. In the case of HRP-conjugated morphine, the addition of a HRP substrate will allow the detection. Colour detection is commonly used but needs incubation time, whereas luminol-derived substances alternatives allow a direct (no incubation time) and a highly sensitive detection.

The concentration of endogenous morphine or a naturally occurring metabolite thereof may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation. A radioimmunoassay for detecting morphine or a naturally occurring metabolite thereof has been commercialized under the name "*COAT-A-COUNT Morphine*" by DPC Diagnostic. ELISA kits for detecting morphine or a naturally occurring metabolite thereof are commercially available from Immunalysis Corporation (Pomona, 91767.California, US) under the reference 213-0192.

Measuring the concentration of morphine or a naturally occurring metabolite thereof (with or without immunoassay-based methods) may also include separation of the compounds: HPLC based on hydrophobicity; size exclusion chromatography based on size; and solid-phase affinity based on the compound's affinity for the particular solid-phase that is used. Once separated, morphine or a naturally occurring metabolite thereof may be identified based on the known "separation profile" e. g., retention time, for that compound and measured using standard techniques. For example, document US Pat No. 4,473,640 discloses a method for detecting morphine comprising hydrolysis with a beta-glucuronidase followed chromatography. Goumon et al. (2005) describe the detection of morphine or a naturally occurring metabolite thereof by reverse phase HPLC.

The separated compounds may be detected and measured by, for example, a mass spectrometer.

Other methods can include the enzymatic detection of morphine or a naturally occurring metabolite thereof. For example, document US 5,298,414 discloses a method based on the enzymatic oxidation of morphine to morphinone by morphine deshydrogenase wherein the enzymatic reaction is coupled to a sensor.

In one embodiment, the method of the invention further may comprise a step of comparing the concentration of endogenous morphine or a naturally occurring metabolite thereof with a predetermined threshold value. Said comparison is indicative of infection. Endogenous morphine is increased during the development of infection.

A further object of the invention relates to a method for detecting sepsis in a patient, said method comprising the steps of :
(i) measuring the concentration of endogenous morphine or a naturally occurring metabolite thereof in a a biological sample obtained from said patient,
(ii) comparing the concentration of endogenous morphine or a naturally occurring metabolite thereof measured in step (i) to a reference value derived from the concentration of endogenous morphine or a naturally occurring metabolite thereof in a biological sample from a subject who does not suffer from infection,
wherein an elevated level of endogenous morphine or a naturally occurring metabolite thereof in the biological sample obtained from said patient as compared to said reference value indicates that the patient suffers from sepsis.

For example, if the biological sample is serum, the level of endogenous morphine or a naturally occurring metabolite thereof in a serum sample can be deemed to be elevated when it is higher than 0.5 ng/ml, preferably higher than 1 ng/ml, even more preferably higher than 1.5 ng/ml.

A further object of the invention relates to the use of endogenous morphine or a naturally occurring metabolite thereof as a marker of sepsis, in a patient.

A further object of the invention relates to the use of a kit detecting endogenous morphine or a naturally occurring metabolite thereof for diagnosing sepsis in a patient.

Yet another object of the invention relates to a kit for detecting sepsis in a patient, comprising a binding partner which selectively interacts with morphine or a naturally occurring metabolite thereof such as an anti-morphine antibody as above described and at least one antibody selected from the group consisting of an anti-CRP antibody and an anti-PCT antibody. The antibody may be labelled as above described. The kit may also contain other suitably packaged reagents and materials needed for the particular detection protocol, including solid-phase matrices, if applicable, and standards.

The method or the kit of the invention can also be used in combination with other methods commonly used for the diagnosis of sepsis in a patient.

Typically, the method of the invention further comprises the step of detecting CRP and/or procalcitonin in a biological sample obtained from said patient.

Typically, the method of the invention further comprises the step of determining the erythrocyte sedimentation.

Typically, the method of the invention further comprises the step of determining the white blood cell count.

The kit contains an anti-CRP antibody and/or an anti-procalcitonin antibody. Typically, the kit can enable the detection of endogenous morphine or a naturally occurring metabolite thereof and the detection of CRP or procalcitonin in a single biological sample. In one embodiment, said sample is divided into two samples for the detection in parallel assays of endogenous morphine or a naturally occurring metabolite thereof on the one hand and the detection of CRP or procalcitonin on the other hand. In one embodiment, the sample will be used for the multiplex measurement of both markers in one single assay. In such an embodiment, the kit contains a binding partner which selectively interacts with endogenous morphine or a naturally occurring metabolite thereof and anti-CRP or anti-procalcitonin antibody which are labelled in a manner that enables their simultaneous detection.

The inventors have demonstrated that endogenous morphine is a marker for sepsis, in contrast with commonly used markers which are elevated in the case of inflammation. Endogenous morphine levels are increased upon any infection, independently of the infectious agent Endogenous morphine, or a naturally occurring metabolite thereof, represents an accurate tool of detecting sepsis in a patient.

The invention will further be illustrated in view of the following figures and example.

### FIGURE LEGENDS

Figure 1 represents the mean ± SEM endogenous morphine concentration measured in the serum of infected and non-infected patients over 3 days of monitoring. *: p<0.05 compared to non-infected patients.
Figure 2 represents the mean ± SEM endogenous morphine concentration measured in the serum of patients with sepsis (S), severe sepsis/septic shock (SS/SSH) compared to healthy patients (No SIRS No Infection, NSNI) over 3 days of monitoring. *: p<0.05 compared to healthy patients.
Figure 3 represents the individual and the mean endogenous morphine concentrations measured in the serum of patients with S, SS and SSH in comparison with healthy donors (NSNI) and patients with SIRS. *: p<0.05 compared to NSNI and SIRS groups.

### EXAMPLE

### Material and Methods :

### Morphine specific ELISA

The morphine specific ELISA kit from Immunalysis Corporation was used for the quantification of endogenous morphine present in secretion and tissue extracts as well as for determination of endogenous morphine plasma levels in septic patients. The specificity of the test for endogenous morphine was confirmed by testing different amount of M6G, M3G and codeine (0-50 ng/ml, data not shown). For all tests, standards of morphine were diluted in the appropriate buffer. 40µl of blood, culture media or serum were tested in duplicates.

### Study setting and population

This study protocol was approved by our institutional review board for human experimentation; written informed consent was obtained before enrolment from each participant or the authorized representative. 100 consecutive patients admitted between July and September 2006 to the intensive care unit (ICU) of the Hospital Hautepierre (Strasbourg, France) were prospectively considered for inclusion. Exclusion criteria comprise former chronic renal, hepatic or cardiac failure (Taupenot, 2003), ongoing steroids (Rozansky, 1994) proton pump inhibitors and a medical history with neuroendocrine tumours (Berruti, 2005). Patients with recent (within 1 month) multiple stress factors, such as repeated surgical interventions, were excluded. Patients who received exogenous morphine before admission or during their stay on ICU were also excluded.

### Data collection

Participants (n=43) were divided into five groups according to ASCCP/SCCM criteria: non septic, non stressed patients (NSNI, comatose patients after self-poisoning, requiring therefore transiently mechanical ventilation; n=5), SIRS (Systemic Inflammatory Response Syndrome) patients (out of hospital cardiac arrest without any exclusion criteria nor infection; n=9), infected patients with criteria of sepsis (S; n=8), severe sepsis (SS; n=6) or septic shock (SSH; n=15). For diagnosis of infection, special attention was paid to fever or hypothermia, laboratory relevant positive cultures and clinical examination according to the definition of specific infection (Levy, 2003). In septic patients (S, SS and SSH), sources of infection were respiratory, urinary or digestive tract. Gram negative bacteria were involved in 55 % of the cases and Gram positive bacteria in 45%. Critically ill patients (SIRS, SSH) were older (p<0.05) than HC. Between groups of patients, there were no differences with regard to mean initial time of first organ dysfunction from admission or mean ICU stay. SIRS and SSH patients underwent significantly more often mechanical ventilation compared with other critically ill patients (p<0.01). SSH patients had significantly higher concentrations of infection biomarkers than healthy controls (p<0.01).

### Statistical analysis of patient results

Data are expressed as mean ± SEM. SPSS was used for statistical analysis of non parametric data, including Mann-Whitney tests, One-Way Anova analysis, Spearman's Correlation test, Fisher Exact test, Repeated-Measure analysis and Receiver Operating Characteristics (ROC) curves comparisons (p<0.05 was considered to be significant).

### Results :

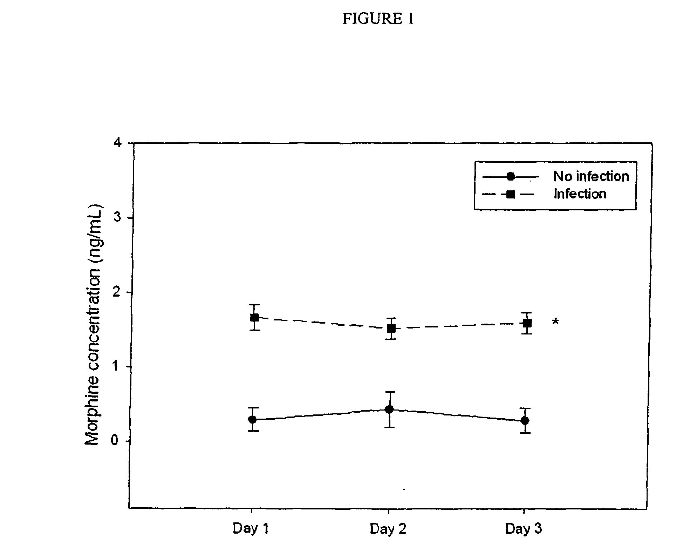

The circulating amount of endogenous morphine was measured in the serum of a cohort of patient of five groups: non septic non stressed patients (NSNI, comatose patients after self-poisoning, requiring therefore transiently mechanical ventilation), SIRS patients (out hospital cardiac arrest without infection), infected patients with criteria of sepsis (S), severe sepsis or septic shock (SS and SSH respectively). Morphine specific ELISA was used to measure the concentration of endogenous morphine present in each serum. Data were first analyzed to determine if a difference exists between serum from infected and non infected patients. This analysis shows that the endogenous morphine concentration found in the serum of infected patients was significantly higher than the concentration found in non infected patients (Fig. 1). This concentration was significantly higher during the three days of monitoring.

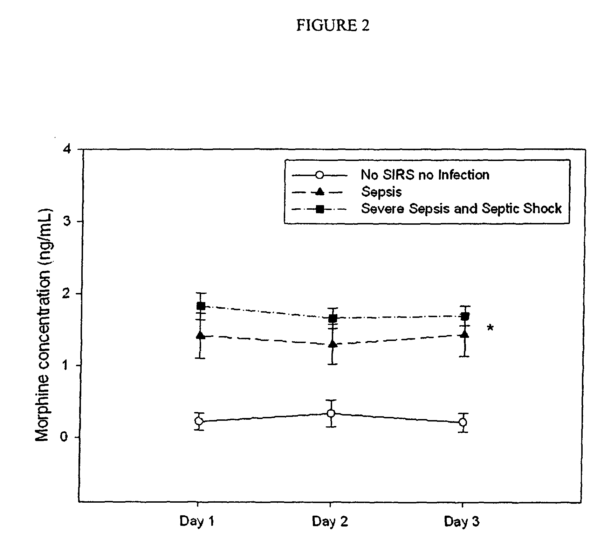

In a second step, data were analyzed in order to determine if a difference exists between healthy donors (no SIRS and no Infection; NSNI), and patients with sepsis (S) and severe sepsis/septic shock (SS/SSH; Fig. 2). This analysis revealed that endogenous concentrations were significantly higher in patients with sepsis and severe sepsis/septic shock than healthy donors, during the three days of monitoring. No significant difference was observed between sepsis and severe sepsis/septic shock groups.

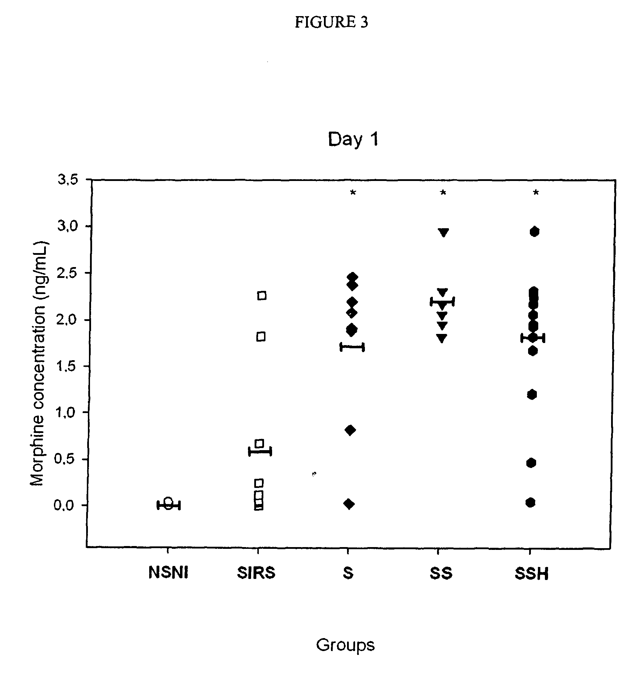

To finish all groups were compared together (day 1) showing that endogenous concentrations increase significantly only in patients with S, SS and SSH in comparison with healthy donors (NSNI) and SIRS (Fig. 3).

Together these data indicate that endogenous morphine represents a marker of sepsis.

### REFERENCES

Berruti A, Mosca A, Tucci M, Terrone C, Torta M, Tarabuzzi R, Russo L, Cracco C, Bollito E, Scarpa RM, Angeli A, Dogliotti L. Independent prognostic role of circulating chromogranin A in prostate cancer patients with hormone-refractory disease. Endocr Relat Cancer. 2005 Mar;12(1):109-17.

Bensousan TA, Vincent F, Assicot M, Morin JF Leclercq B, Escudier B, Nitenberg G. Evolution des cytokines, de la procalcitonine (ProCT) et des peptides opioïde au cours d'un modèle clinique de syndrome inflammatoire (SlRS). Reanimation Urgences. 1996 5(6): 749

Colas P, Cohen B, Jessen T, Grishina I, McCoy J, Brent R. (1996) Genetic selection of peptide aptamers that recognize and inhibit cyclin-dependent kinase 2. Nature, 380, 548-50.

Cote RJ, Morrissey DM, Houghton AN, Beattie EJ Jr, Oettgen HF, Old LJ. Generation of human monoclonal antibodies reactive with cellular antigens. Proc Natl Acad Sci USA. 1983 Apr;80(7):2026-30.

Goumon Y, Strub JM, Stefano GB, Van Dorsselaer A, Aunis D, Metz-Boutigue MH. Characterization of a morphine-like molecule in secretory granules of chromafifn cells. Med Sci Monit. 2005 11 (5): MS31-34.

Goumon Y, Muller A, Glattard E, Marban C, Gasnier C, Strub JM, Chasserot-Golaz S, Rohr O, Stefano GB, Welters I, Van Dorsselaer A, Schoentgen F, Aunis D, Metz-Boutigue MH. Identification of morphine-6-glucuronide in chromaffin cell secretory granules. JBC 2006 281 (12): 8082-89.

Kohler G, Milstein C. Continuous cultures of fused cells secreting antibody of predefined specificity. Nature. 1975 Aug 7;256(5517):495-7.

Levy MM, Fink MP, Marshall JC, Abraham E, Angus D, Cook D, Cohen J, Opal SM, Vincent JL, Ramsay G; International Sepsis Definitions Conference. 2001 SCCM/ESICM/ACCP/ATS/SIS International Sepsis Definitions Conference. Intensive Care Med. 2003 Apr;29(4):530-8. Epub 2003 Mar 28. Review.

Martin GS, Mannino DM, Eaton S, et al. The epidemiology of sepsis in the United States from 1979 through 2000. N Engl J Med 2003; 348:1546-54. Reinhart K, Meisner M, Brunkhorst FM. Markers for sepsis diagnosis: what is useful? Crit Care Clin 2006 Jul 22(3): 503-19.

Rozansky DJ, Wu H, Tang K, Parmer RJ, O'Connor DT. Glucocorticoid activation of chromogranin A gene expression. Identification and characterization of a novel glucocorticoid response element. J Clin Invest. 1994 Dec;94(6):2357-68.

Silverman MN, Pearce BD, Biron CA, et al. Immune modulation of the hypothalamic-pituitary-adrenal (HPA) axis during viral infection. Viral Immunol 2005; 18: 41-78.

Singer M, De Santis V, Vitale D, et al. Multiorgan failure is an adaptive, endocrine-mediated, metabolic response to overwhelming systemic inflammation. Lancet 2004; 364: 545-48.

Spapen HD, Hachimi-ldrissi S, Corne L, Huyghens LP. Diagnostic markers of sepsis in the emergency department. Acta Clin Belg 2006, 61 (3) 138-42.

Stefano GB, Goumon Y, Casares F, Cadet P, Fricchione GL, Rialas C, Doris P, Sonetti D, Guarna M, Welters ID, Bianchi E. Endogenous morphine. TINS2000, 23(9): 436-442.

Taupenot L, Harper KL, O'Connor DT. The chromogranin-secretogranin family. N Engl J Med. 2003 Mar 20; 348(12):1134-49.

Tsiotou AD, Sakorafas, GH, Anagnostopoulos G, Bramis J. Septic shock; current pathogenetic concepts from a clinical perspective. Med Sci Monit 2005; 11 (3): RA76-85.

Tuerk C., Using the SELEX combinatorial chemistry process to find high affinity nucleic acid ligands to target molecules. Methods Mol Biol.1997; 67: 219-30.

## Claims

1. A method for detecting sepsis in a patient, said method comprising detecting endogenous morphine or a naturally occurring metabolite thereof in a biological sample obtained from said patient.

2. A method for detecting sepsis in a patient, said method comprising measuring the concentration of endogenous morphine or a naturally occurring metabolite thereof in a biological sample obtained from said patient.

3. The method according to any one of claims 1 to 2, wherein said naturally occurring metabolite thereof compound is morphine-6-glucuronide (M6G).

4. The method according to any one of claims 1 to 3, wherein said biological sample is a blood sample, a serum sample, a plasma, or a urine sample.

5. Use of endogenous morphine or a naturally occurring metabolite thereof as an in vitro marker of sepsis in a patient.

6. Use of a kit detecting endogenous morphine or a naturally occurring metabolite thereof for in vitro diagnosing sepsis in a patient.

7. A kit comprising a binding partner which selectively interacts with endogenous morphine or a naturally occurring metabolite thereof and at least one antibody selected from the group consisting of an anti-C-reactive Protein (CRP) antibody and an anti-procalcitonin antibody.

8. A kit according to claim 7 wherein said binding partner which selectively interacts with endogenous morphine or a naturally occurring metabolite thereof is an anti-morphine antibody.

## Patentansprüche

1. Verfahren zum Feststellen von Sepsis in einem Patienten, wobei das Verfahren umfasst, endogenes Morphin oder einen natürlich vorkommenden Metaboliten davon in einer von dem Patienten erhaltenen biologischen Probe nachzuweisen.

2. Verfahren zum Feststellen von Sepsis in einem Patienten, wobei das Verfahren umfasst, die Konzentration von endogenem Morphin oder einem natürlich vorkommenden Metaboliten davon in einer von dem Patienten erhaltenen biologischen Probe zu messen.

3. Verfahren gemäß einem der Ansprüche 1 bis 2, wobei die Verbindung, die der natürlich vorkommende Metabolit davon ist, Morphin-6-glucuronid (M6G) ist.

4. Verfahren gemäß einem der Ansprüche 1 bis 3, wobei die biologische Probe eine Blutprobe, eine Serumprobe, ein Plasma oder eine Urinprobe ist.

5. Verwendung von endogenem Morphin oder einem natürlich vorkommenden Metaboliten davon als in vitro-Marker für Sepsis in einem Patienten.

6. Verwendung eines Kits, welches endogenes Morphin oder einen natürlich vorkommenden Metaboliten davon nachweist, um Sepsis in einem Patienten in vitro zu diagnostizieren.

7. Kit, umfassend einen Bindungspartner, der selektiv mit endogenem Morphin oder einem natürlich vorkommenden Metaboliten davon wechselwirkt, und mindestens einen Antikörper, ausgewählt aus der Gruppe bestehend aus einem Anti-C-reaktives Protein- (CRP) Antikörper und einem Anti-Procalcitonin-Antikörper.

8. Kit gemäß Anspruch 7, wobei der Bindungspartner, der selektiv mit endogenem Morphin oder einem natürlich vorkommenden Metaboliten davon wechselwirkt, ein Anti-Morphin-Antikörper ist.

## Revendications

1. Procédé de détection d'une septicémie chez un patient, ledit procédé comprenant la détection d'une morphine endogène ou d'un métabolite naturel de celle-ci dans un échantillon biologique obtenu chez ledit patient.

2. Procédé de détection d'une septicémie chez un patient, ledit procédé comprenant la mesure de la concentration d'une morphine endogène ou d'un métabolite naturel de celle-ci dans un échantillon biologique obtenu chez ledit patient.

3. Procédé selon l'une quelconque des revendications 1 à 2, dans lequel ledit métabolite naturel de celle-ci est la morphine-6-glucuronide (M6G).

4. Procédé selon l'une quelconque des revendications 1 à 3, dans lequel ledit échantillon biologique est un échantillon de sang, un échantillon de sérum, un plasma ou un échantillon d'urine.

5. Utilisation d'une morphine endogène ou d'un métabolite naturel de celle-ci en tant que marqueur *in vitro* de septicémie chez un patient.

6. Utilisation d'un kit détectant une morphine endogène ou un métabolite naturel de celle-ci pour diagnostiquer *in vitro* une septicémie chez un patient.

7. Kit comprenant un partenaire de liaison qui interagit sélectivement avec une morphine endogène ou un métabolite naturel de celle-ci et au moins un anticorps choisi dans le groupe constitue par un anticorps anti-protéine C réactive (CRP) et un anticorps anti-procalcitonine.

8. Kit selon la revendication 7, dans lequel ledit partenaire de liaison qui interagit sélectivement avec une morphine endogène ou un métabolite naturel de celle-ci est un anticorps anti-morphine.
